(19) Europäisches Patentamt
European Patent Office
Office européen des brevets

(11) Publication number: **0 013 768**
**B1**

(12) **EUROPEAN PATENT SPECIFICATION**

(45) Date of publication of patent specification: **01.02.84**

(21) Application number: **79105421.6**

(22) Date of filing: **31.12.79**

(51) Int. Cl.³: **C 07 D 249/12,**
A 61 K 31/41,
C 07 D 249/14,
C 07 D 401/04,
A 61 K 31/44 //(C07D401/04,
249/08, 213/00)

(54) **New pleuromutilin derivatives, their production and pharmaceutical compositions containing them.**

(30) Priority: **12.01.79 CH 309/79**
**08.08.79 CH 7274/79**

(43) Date of publication of application:
**06.08.80 Bulletin 80/16**

(45) Publication of the grant of the patent:
**01.02.84 Bulletin 84/5**

(84) Designated Contracting States:
**AT BE CH DE FR GB IT LU NL SE**

(56) References cited:
**GB - A - 1 312 148**

**THE JOURNAL OF ANTIBIOTICS, vol. XXIX, no. 9, published September 1976, pages 915-922 Tokyo, JP. H. EGGER et al.: "New pleuromutilin derivatives with enhanced antimicrobial activity. I. Synthesis"**
**THE JOURNAL OF ANTIBIOTICS, vol. XXIX, no. 9, published September 1976, pages 923-927 Tokyo, JP. H. EGGER et al.: "New pleuromutilin derivatives with enhanced antimicrobial activity. II. Structure-activity correlations"**

(73) Proprietor: **SANDOZ AG**
**Lichtstrasse 35**
**CH-4002 Basel (CH)**

(72) Inventor: **Berner, Heinz, Dr.**
**Martinstrasse 83**
**A-1180 Wien (AT)**
Inventor: **Turnowsky, Friederike, Dr.**
**Wienerbruckstrasse 87**
**A-2344 Maria Enzersdorf (AT)**
Inventor: **Laber, Georg, Dr.**
**Alserstrasse 18/30**
**A-1090 Wien (AT)**
Inventor: **Hildebrandt, Johannes, Dr.**
**St. Laurentgasse 12**
**A-2512 Oeynhausen (AT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 013 768 B1**

# New pleuromutilin derivatives, their production and pharmaceutical compositions containing them

This invention provides compounds of formula I

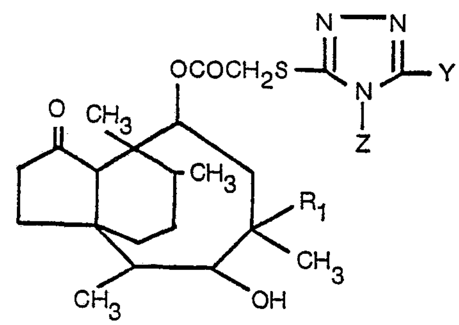

I

in which

$R_1$ is ethyl or vinyl;

Y is hydrogen, amino, trifluoromethyl, $C_1$—$C_4$-alkyl, pyridyl or —$N(R_3)(R_4)$;

Z is hydrogen, $C_1$—$C_4$-alkylsulphonyl, amino, formyl, —$N(R_3)(R_4)$, —$S(CH_2)_nN(R_3)(R_4)$ or $CX.NHR_6$;

$R_3$ and $R_4$ are the same or different and each is hydrogen, $C_1$—$C_4$-hydroxyalkyl, $C_1$—$C_4$-dihydroxyalkyl, unsubstituted or substituted $C_1$—$C_4$-alkanoyl, $C_1$—$C_4$-alkylsulphonyl or $C_1$—$C_4$-alkyl or together with the nitrogen atom stand for a piperazinyl radical which may be substituted on the second nitrogen atom by $C_1$—$C_4$-alkyl, $C_1$—$C_4$-hydroxyalkyl or $C_1$—$C_4$-dihydroxyalkyl;

$R_6$ is $C_1$—$C_4$-alkyl or $C_1$—$C_4$-alkoxy-carbonyl;

n is 2 to 5; and

x is oxygen or sulphur;

and acid addition and quaternary ammonium salts thereof.

Egger et. al. J. Antibiotics, v. XXIV, No. 9, pp. 923—27 describe antimicrobial pleuromutilin derivatives having a side chain and bearing i.a. saturated heterocyclic groups attached via a heteroatom to said side chain. There is no disclosure of compounds of the present invention.

The invention also provides a process for the production of compounds of formula I, characterised by reacting a compound of formula II,

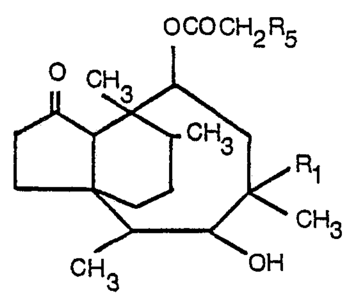

II

in which

$R_1$ is as defined above, and

$R_5$ is chlorine, bromine or —$OSO_2R_7$, in which $R_7$ is alkyl or aryl,

with a compound of formula III,

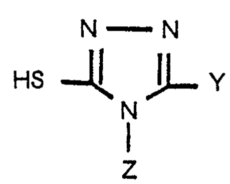

III

in which Y and Z are as defined above.

The process is suitably carried out in the presence of an alkali metal lower alkoxide, for example sodium ethoxide or methoxide. This is preferably produced *in situ*. Conveniently, the compound of formula III may be dissolved in a solution of sodium in a water-free lower alkanol, e.g. methanol or ethanol. A solution of the compound of formula II in an inert organic solvent, e.g. an aliphatic ketone,

such as methyl ethyl ketone or acetone, is then conveniently added. The process is suitably effected at a temperature from room temperature to the reflux temperature of the reaction mixture, in particular from 22° to 55°C. The reaction time may typically vary from 2 to 12 hours.

The resulting compounds of formula I may be isolated and purified using conventional techniques. Where required, free base forms thereof may be converted into salt forms, in particular into acid addition salt and quaternary ammonium salt forms, in conventional manner, and *vice versa*.

The compounds of formula I are indicated for use as chemotherapeutic agents, in particular as antimicrobial agents, as indicated, e.g. by their inhibiting effect against various bacterial strains, e.g. Staph. aureus, Staph. epidermis, Strept. pyogenes, Strept. aranson, Strept. pneumoniae, Strept. faecelis, Strept. viridans, Corynebact. pyogenes, Sarcina lutea, Klebsiella pneumoniae, and Haemophilus influenzae, *in vitro* in the series dilution test at a concentration, for example, of 0.01 to 25 ug/ml, and in *in vivo* tests in mice. The compounds also show an inhibiting effect against various mycoplasma, e.g. M. hominis, M. arthritidis, M. pneumoniae, and urea plasma urealyticum, and chlamydia, *in vitro* in the series dilution test at concentrations of, for example, 0.008 to 2.5 ug/ml.

The compounds also show an inhibiting effect against various obligatory anaerobes, e.g. Bacteroides fragilis, Bacteroides melaninogenicus, Sphaerophorus necrophorus, Clostridium perfringens, etc., *in vitro* in the series dilution test at concentrations of for example 0.1 to 4 ug/ml, and *in vivo* in mice at a dosage of for example 50 to 200 mg/kg of animal body weight, p.o. or s.c.

The compounds are therefore indicated for use as anti microbial agents, in particular as antibacterially active antibiotics and for treatment of infections caused by obligatory anaerobes.

For the above-mentioned uses, an indicated suitable daily dosage is from about 1 to 3 g, suitably administered in divided dosages of from two to four times daily, or in retard form.

The compounds alone or in admixture with a tetracyclin may be administered orally or parenterally in such forms as tablets, capsules, powders, granulates, and injectable or infusion preparations, e.g. solutions or suspensions. The compounds may also be employed in the form of creams or tinctures. For veterinary purposes, the compounds may also be administered as food or drink additives.

It has also been found that mixtures of the compounds of formula I with a tetracyclin show a synergistic antibacterial effect against strains with R-factor coded tetracyclin resistence. This indicated for example by determination of the minimum inhibition concentration of the mixture and the individual components in the series dilution test, and by evaluating the results by the method of Löwe (isobole diagram), Die Antibiotika, Volume 1, part 1, 65ff, 1962. Conventional tetracyclines, e.g. chlorotetracyclin, oxytetracyclin, demethyltetracyclin, tetracyclin dioxycyclin, monocyclin, metacyclin, and rolitetracyclin, may be employed in such mixtures. The quantity of the compound of formula I in such mixtures is suitably 10 to 90%, preferably 20 to 35%, in particular 25%, while the quantity of the tetracyclin is suitably from 90 to 10%, preferably 80 to 65%, particularly 75% (these percentages being by weight).

The mixtures are particularly indicated in treating infections of the gastrointestinal tract and other local infections of the organ.

The compounds of formula I, when used alone or in admixture with a tetracyclin, may be employed in free base form or in the form of chemotherapeutically acceptable acid addition or quaternary ammonium salts. These salt forms have the same order of activity as the free base forms.

Suitable acid addition salt forms include the hydrochloride, hydrogen, fumarate, fumarate and naphthalene-1,5-sulphonate.

The compounds (or mixtures thereof with a tetracycline) may be admixed with a chemotherapeutically acceptable diluent or carrier and, optionally other conventional excipients for the production of galenic forms. Suitable excipients include sweeteners, aromas, colouring agents, preserving agents, e.g. ethyl-*o*-hydroxybenzoate, fillers or carriers, e.g. diluents, such as calcium carbonate, disintegrating agents, e.g. starch or alginic acid, binding agents, e.g. starch, gelatine or acacia, and lubricating agents, e.g. magnesium stearate, stearic acid or talc. Oral liquid forms may contain conventional suspending agents, e.g. methylcellulose, tragacanth or sodium alginate. Suitable wetting agents include lecithin, polyoxyethane stearate and polyoxyethylene sorbitan monooleate. For the production of capsules, suitable diluents include calcium carbonate, calcium phosphate and kaolin.

The preferred compound of formula I is that of Example I, hereinafter.

The following Examples illustrate the invention. All temperatures are in degrees Centigrade.

Example 1

19,20-Dihydro-14-O-[(3-amino-1,2,4-triazol-5-yl)thioacetyl]mutilin

2.3 g of sodium are taken up in 500 ml of absolute ethanol. After formation of the sodium ethoxide, 11.6 g of 3-amino-5-mercapto-1,2,4-triazol are added to the solution.

The mixture is allowed to react for 3 hours at 25°C and is then mixed with a solution of 53.5 g of 19,20-dihydro-22-O-tosyl-pleuromutilin in 200 ml of ethylmethylketone. The homogenous reaction mixture is held for 12 hours at 25° and then poured onto water and extracted 3 times with 500 ml of ethyl acetate. The purified ethyl acetate extract is shaken with water, dried over $Na_2SO_4$ and evaporated

*in vacuo*. The crude product is chromatographed over silica gel (eluant: ethyl acetate) to obtain the heading compound, m.p. 213—215° (isopropanol/$H_2O$).

NMR (DMSO): 5.76 (broad, 2H, $NH_2$); 5.52 (d, 1H, $H_{14}$, $J_{H_{14}H_{13}} = 8$ Hz); 3.76 (s, 2H, S—$CH_2$—CO); 3.35 (m, 1H, $H_{11}$).

IR (—KBr): 2600—3600 (broad), 1720, 1635, 1280 $cm^{-1}$.

The compounds of the following Examples may be produced in manner analogous to that of Example 1, using appropriate starting materials in approximately equivalent amounts.

Example 2

14-O-[(3-Amino-1,2,4-triazol-5-yl)thioacetyl]-mutilin

NMR ($CDCl_3$): 5.68 (d, 1H, $H_{14}$, $J_{H_{14}H_{13}} = 8$ Hz), 3.34 (d, 1H, $H_{11}$, $J_{H_{11}H_{10}} = 6$ Hz); 3.7 (s, 2H, S—$CH_2$—CO); 5.26—4.95 (m, 4H, $NH_2 + 2H_{20}$); 6.5—6.16 (m, 1H, $H_{19}$).

IR (KBr): 3300 (broad), 1720, 1625, 1575, 1270 $cm^{-1}$.

Example 3

14-O-[(4-Methylsulfonyl-5-amino-1,2,4-triazol-3-yl)-thioacetyl]mutilin

NMR ($CDCl_3$): 5.9 (s, 2H, $NH_2$); 5.76 (di, 1H, $H_{14}$, $J_{H_{14}H_{13}} = 8$ Hz); 3.81 (s, 2H, S—$CH_2$—CO); 3.3 (s, 3H, $CH_3SO_2$—); 3.4 (m, 1H, $H_{11}$).

IR (KBr): 3400 (broad), 1720, 1625, 1265, 1275, 1180 $cm^{-1}$.

Example 4

14-O-[(4-Amino-1,2,4-triazol-3-yl)thioacetyl]mutilin

NMR ($CDCl_3$): 8.26 (s, 1H, Triazol-H); 5.7 (d, 1H, $H_{14}$, $J_{H_{14}H_{13}} = 8$ Hz); 5.08 (s, 2H, $NH_2$), AB-System ($v_A = 3.86$, $v_B = 3.75$, $J_{AB} = 16.2$ Hz, S—$CH_2$—O); 3.34 (dd, 1H, $H_{11}$, J=6.3 Hz, J=10.2 Hz).

IR (KBr): 3400 (broad), 1720 $cm^{-1}$.

Example 5

14-O-[(3-(4-Pyridyl)-1,2,4-triazol-5-yl)thioacetyl]-mutilinhydrochlorid

NMR (DMSO): 8.95 (d, 2H, Pyridin-H, J=6.3 Hz); 8.36 (d, 2H-Pyridin-H, J=6.3 Hz); 5.55 (d, 1H, $H_{14}$, $J_{H_{14}H_{13}} = 8$ Hz); 4.16 (s, 2H, S—$CH_2$—CO); 3.4 (d, 1H, $H_{11}$, $J_{H_{10}H_{11}} = 6.3$ Hz).

IR (KBr): 3600—2500 (broad), 1725, 1635 $cm^{-1}$.

Example 6

14-O-[(4-Amino-3-trifluoromethyl-1,2,4-triazol-5-yl)-thioacetyl]mutilin

NMR ($CDCl_3$): 5.74 (d, 1H, $H_{14}$, $J_{H_{14}H_{13}} = 8$ Hz); 5.18 (s, 2H, $NH_2$); 3.9 (s, 2H, S—$CH_2$CO); 3.38 (1H, $H_{11}$).

IR (KBr): 3400 (broad), 1720, 1190, 1150 $cm^{-1}$.

Example 7

14-O-[(4-Amino-3-methyl-1,2,4-triazol-5-yl)thioacetyl]mutilin

NMR ($CDCl_3$): 5.72 (d, 1H, $H_{14}$, $J_{H_{14}H_{13}} = 8$ Hz); 4.97 (s, 2H, $NH_2$); AB-System ($v_A$=3.84, $v_B$=3.69, $J_{AB}$=16.2 Hz, S—$CH_2$—CO); 3.38 (dd, $H_{11}$, J=6.3 Hz, J=10.2 Hz).

IR (KBr): 3400 (broad), 1725 $cm^{-1}$.

Example 8

14-O-[(3-Methyl-4-acetamido-1,2,4-triazol-5-yl)-thioacetyl]mutilin

NMR ($CDCl_3$): 5.7 (d, 1H, $H_{14}$, $J_{H_{14}H_{13}} = 8$ Hz); 3.8 (s, 2H, S—$CH_2$—CO); 3.38 (m, 1H, $H_{11}$); 2.33 (s, 3H, $CH_3$CO—N); 2.26 (s, 3H, Triazol-$CH_3$).

IR (KBr): 3400 (broad), 1720, 750 $cm^{-1}$.

Example 9

14-O-[(3-(Methoxysulfonylethylcarboxamido)-1,2,4-triazol-5-yl)thioacetyl]mutilin

NMR ($CDCl_3$): 5.72 (d, 1H, $H_{14}$, $J_{H_{14}H_{13}} = 8$ Hz); 3.82 (s, 2H, S—$CH_2$—CO); 3.34 (m, 1H, $H_{11}$); s, 14 (s, 3H, —O—$CH_3$).

IR (KBr): 3400 (broad), 1720, 1625, 1550, 1305, 1110, 730 $cm^{-1}$.

Example 10

14-O-[(1-Ethylaminocarbonyl-3-amino-1,2,4-triazol-5-yl)thioacetyl]mutilin

NMR ($CDCl_3$)/$D_3OD$ 10:1): 5.74 (d, 1H, $H_{14}$, $J_{H_{14}H_{13}} = 8$ Hz); 3.75 (s, 2H, S—$CH_2$—CO); 3—38 (q, 2H, *$CH_3$*—$CH_2$—N); 3.4 (m, 1H, $H_{11}$); 1.26 (t, 3H, *$CH_3$*—$CH_2$—N).

IR (KBr): 3540, 3430, 3310, 1710, 1630, 1300 $cm^{-1}$.

m.p. 230—232°.

Example 11

14-O-[(3-Amino-4-formyl-1,2,4-triazol-5-yl-thioacetyl]mutilin

NMR ($CDCl_3$): 8.52 (s, 1H), Formyl-H); 5.74 (d, 1H, $H_{14}$, $J_{H_{14}H_{13}} = 8$ Hz); 3.82 (s, 2H, S—$CH_2$CO); 3.36 (m, 1H, $H_{11}$).

IR (KBr): 3600—2800 (broad), 1725, 1585, 1290 $cm^{-1}$.

Example 12

14-O-[(3-Amino-1-(carbethoxythiocarbamyl)-1,2,4-triazol-5-yl]thioacetyl]mutilin

NMR ($CDCl_3$): 7.74 (b, 2H, $NH_2$); 5.81 (d, 1H, $H_{14}$, $J_{H_{14}H_{13}} = 8$ Hz); 4.37 (q, 2H, O—$CH_2CH_3$); 3.83 (s, 2H, S—$CH_2$—CO); 3.4 (d, 1H, $H_{11}$, $J_{H_{11}H_{10}} = 6.3$ Hz); 1.38 (t, 3H, O—$CH_2CH_3$).

IR (KBr): 3300 (broad), 1770, 1725, 1635, 1465, 1185 $cm^{-1}$.

Example 13

14-O-[(3-Amino-4-(Ethylaminothiocarbonyl)-1,2,4-triazol-5-yl)thioacetyl]mutilin

NMR ($CDCl_3$): 8.56 (m, 1H, NH); 7.4 (b, 2H, $NH_2$); 5.76 (d, 1H, $H_{14}$, $J_{H_{14}H_{13}} = 8$ Hz); 3.78 (s, 2H, S—$CH_2$—CO); 3.68 (m, 2H, N—$CH_2$—$CH_3$); 3.38 (dd, 1H, $H_{11}$, J=6.3 Hz, J=10.2 Hz); 1.33 (t, 3H, N—$CH_2$—$CH_3$).

IR (KBr): 3340 (broad), 1720, 1630, 1290 $cm^{-1}$.

Example 14

14-O-[(4-Bis-(methylsulfonylamino)-1,2,4-triazol-3-yl]mutilin

NMR ($CDCl_3$): 8.28 (s, 1H, Triazol-H); 5.72 (d, 1H, $H_{14}$, $J_{H_{14}H_{13}} = 8$ Hz); AB-System ($v_A$=4.18, $v_B$=4.02, $J_{AB}$=16.2 Hz, S—$CH_2$—CO); 3.6 (s, 3H, $CH_3SO_2$—); 3.58 (s, 3H, $CH_3SO_2$—); 3.34 (m, 1H, $H_{11}$).

IR (KBr): 3450 (broad), 1720, 1380, 1160 $cm^{-1}$.

Example 15

14-O-[(3-(2-Pyridyl)-1,2,4-triazol-5-yl]thioacetyl]mutilin

NMR ($CDCl_3$): 8.82 (d, 1H, Pyridin-H, J=5 Hz); 8.24 (d, 1H, Pyridin-H, J=10 Hz); 7.9 (m, 1H, Pyridin-H); 7.45 (m, 1H, Pyridin-H); 5.78 (d, 1H, $H_{14}$, $J_{H_{14}H_{13}} = 8$ Hz); 4.0 (s, 2H, S—$CH_2$—CO); 3.4 (m, 1H, $H_{11}$).

IR (KBr): 3500—2800 (broad), 1720, 1450, 1280 $cm^{-1}$.

UV ($CH_3CH$): 232 nm ($\Sigma = 12400$), 282 (8170).

**Claims for the Contracting States: BE CH DE FR GB IT LU NL SE**

1. A compound of formula I,

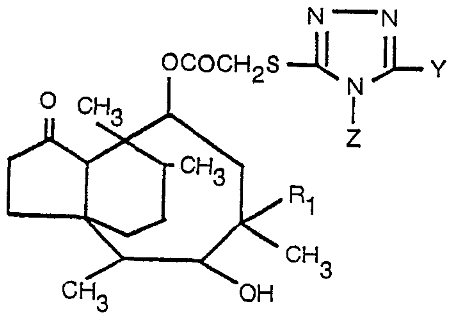

I

in which

$R_1$ is ethyl or vinyl;

Y is hydrogen, amino, trifluoromethyl, $C_1$—$C_4$-alkyl, pyridyl or —$N(R_3)(R_4)$;

Z is hydrogen, $C_1$—$C_4$-alkylsulphonyl, amino, formyl, —$N(R_3)(R_4)$, —$S(CH_2)_nN(R_3)(R_4)$ or CX.$NHR_6$;

$R_3$ and $R_4$ are the same or different and each is hydrogen, $C_1$—$C_4$-hydroxyalkyl, $C_1$—$C_4$-dihydroxyalkyl, unsubstituted or substituted $C_1$—$C_4$-alkanoyl, $C_1$—$C_4$-alkylsulphonyl or $C_1$—$C_4$-alkyl or together with the nitrogen atom stand for a piperazinyl radical which may be substituted on the second nitrogen atom by $C_1$—$C_4$-alkyl, $C_1$—$C_4$-hydroxyalkyl or $C_1$—$C_4$-dihydroxyalkyl;

$R_6$ is $C_1$—$C_4$-alkyl or $C_1$—$C_4$-alkoxy-carbonyl;

n is 2 to 5; and

x is oxygen or sulphur;

and acid addition and quaternary ammonium salts thereof.

2. 19,20-Dihydro-14-O-[3-amino-1,2,4-triazole-5-yl)-thioacetyl]mutilin and acid addition and quaternary ammonium salts thereof.

3. A chemotherapeutic composition comprising a compound of formula I as claimed in Claim 1 or 2, in free base form or in the form of a chemotherapeutically acceptable acid addition or quaternary ammonium salt, together with a chemotherapeutically acceptable diluent or carrier.

4. A compound of Formula I as claimed in Claim 1 or 2, in free base form or in the form of a chemotherapeutically acceptable acid addition or quaternary ammonium salt for use as a chemotherapeutic agent.

5. A compound of Formula I as claimed in Claim 1 or 2, in free base form or in the form of a chemotherapeutically acceptable acid addition or quaternary ammonium salt, for use as an antibacterially active antibiotic and for the treatment of infections caused by obligatory anaerobes.

6. A process for the production of a compound of Formula I, stated in Claim 1, or an acid addition or quaternary ammonium salt thereof, which comprises reacting a compound of Formula II,

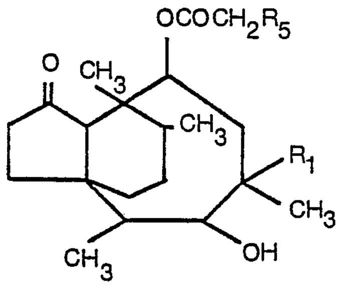

II

in which
$R_1$ is as defined in Claim 1 and
$R_5$ is chlorine, bromine or $—OSO_2R_7$, in which $R_7$ is alkyl or aryl,
with a compound of formula III,

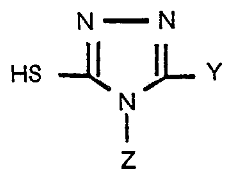

III

in which
Y and Z are as defined in Claim 1, and, if desired, converting a compound of Formula I thus obtained into an acid addition or quaternary ammonium salt thereof or vice versa.

**Claims for the Contracting State: AT**

1. A process for the production of a compound of formula I

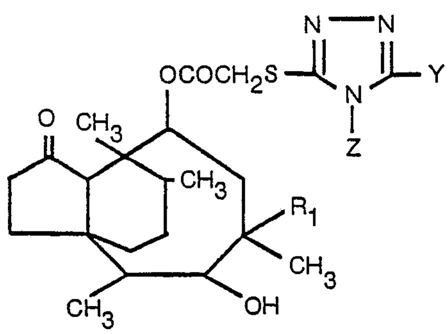

I

in which
$R_1$ is ethyl or vinyl;
Y is hydrogen, amino, trifluoromethyl, $C_1—C_4$-alkyl, pyridyl or $—N(R_3)(R_4)$;
Z is hydrogen, $C_1—C_4$-alkylsulphonyl, amino, formyl, $—N(R_3)(R_4)$, $—S(CH_2)_nN(R_3)(R_4)$ or $CX.NHR_6$;
$R_3$ and $R_4$ are the same or different and each is hydrogen, $C_1—C_4$-hydroxyalkyl, $C_1—C_4$-dihydroxyalkyl, unsubstituted or substituted $C_1—C_4$-alkanoyl, $C_1—C_4$-alkylsulphonyl or $C_1—C_4$-alkyl or together with the nitrogen atom stand for a piperazinyl radical which may be substituted on the second nitrogen atom by $C_1—C_4$-alkyl, $C_1—C_4$-hydroxyalkyl or $C_1—C_4$-dihydroxyalkyl;

$R_6$ is $C_1$—$C_4$-alkyl or $C_1$—$C_4$-alkoxy-carbonyl;
n is 2 to 5; and
x is oxygen or sulphur;

or an acid addition or quaternary ammonium salt thereof, which comprises reacting a compound of formula II,

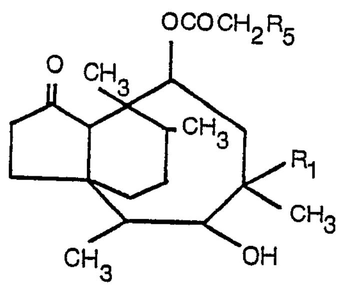

II

in which

$R_1$ is as defined above, and
$R_5$ is chlorine, bromine or —$OSO_2R_7$, in which $R_7$ is alkyl or aryl,

with a compound of formula III,

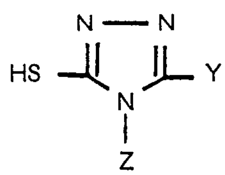

III

in which

Y and Z are as defined above,.and, if desired, converting a compound of formula I thus obtained into an acid addition or quaternary ammonium salt thereof or vice versa.

2. A process as claimed in Claim 1 wherein the compound thus obtained is 19,20-Dihydro-14-O-[3-amino-1,2,4-triazole-5-yl)-thioacetyl]-mutilin or an acid addition or quaternary ammonium salt thereof.

3. A compound of formula I according to Claim 1 or an acid addition or quaternary ammonium salt thereof wherever prepared by a process as claimed in Claim 1.

4. A chemotherapeutic composition comprising a compound of formula I according to Claim 3, in free base form or in the form of a chemotherapeutically acceptable acid addition or quaternary ammonium salt, together with a chemotherapeutically acceptable diluent or carrier.

5. A process for preparing a chemotherapeutic composition which comprises admixing a compound of formula I

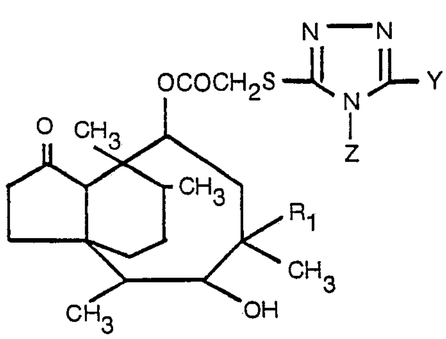

I

in which

$R_1$ is ethyl or vinyl;
Y is hydrogen, amino, trifluoromethyl, $C_1$—$C_4$-alkyl, pyridyl or —$N(R_3)(R_4)$;
Z is hydrogen, $C_1$—$C_4$-alkylsulphonyl, amino, formyl, —$N(R_3)(R_4)$, —$S(CH_2)_nN(R_3)(R_4)$ or $CX.NHR_6$;
$R_3$ and $R_4$ are the same or different and each is hydrogen, $C_1$—$C_4$-hydroxyalkyl, $C_1$—$C_4$-dihydroxyalkyl, unsubstituted or substituted $C_1$—$C_4$-alkanoyl, $C_1$—$C_4$-alkylsulphonyl or $C_1$—$C_4$-alkyl or together with the nitrogen atom stand for a piperazinyl radical which may be substituted on the second nitrogen atom by $C_1$—$C_4$-alkyl, $C_1$—$C_4$-hydroxyalkyl or $C_1$—$C_4$-dihydroxyalkyl;

$R_6$ is $C_1$—$C_4$-alkyl or $C_1$—$C_4$-alkoxy-carbonyl;
n is 2 to 5; and
x is oxygen or sulphur;
in free base form or in the form of a chemotherapeutically acceptable acid addition or quaternary ammonium salt, together with a chemotherapeutically acceptable diluent or carrier.

**Patentansprüche für die Vertragsstaaten: BE CH DE FR GB IT LU NL SE**

1. Eine Verbindung der Formel I

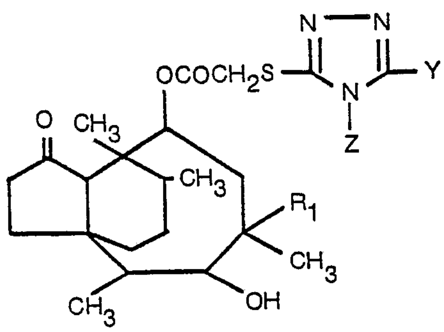

I

worin
$R_1$ Ethyl oder Vinyl;
Y Wasserstoff, Amino, Trifluormethyl, $C_{1-4}$-Alkyl, Pyridyl oder —$N(R_3)(R_4)$
Z Wasserstoff, $C_{1-4}$-Alkylsulfonyl, Amino, Formyl, —$N(R_3)(R_4)$, —$S(CH_2)_n$—, —$N(R_3)(R_4)$ oder CX.NHR$_6$; bedeuten,
$R_3$ und $R_4$ gleich oder verschieden sind und jeweils für Wasserstoff, $C_{1-4}$-Hydroxyalkyl, $C_{1-4}$-Dihydroxyalkyl, unsubstituiertes oder substituiertes $C_{1-4}$-Alkanoyl, $C_{1-4}$-Alkylsulfonyl oder $C_{1-4}$-Alkyl stehen, oder zusammen mit dem Stickstoffatom für einen Piperazinrest stehen der am zweiten Stickstoffatom durch $C_{1-4}$-Alkyl, $C_{1-4}$-Hydroxyalkyl oder $C_{1-4}$-Dihydroxyalkyl substituiert sein kann;
$R_6$ $C_{1-4}$-Alkyl oder $C_{1-4}$-Alkoxy-Carbonyl;
n 2 bis 5; und
X Sauerstoff oder Schwefel sind,
und deren Säureadditions- und quaternäre Ammoniumsalze.

2. 19,20-Dihydro-14-*O*-[3-amino-1,2,4-triazol-5-yl)-thioacetyl]-mutilin und Säureadditions- und quaternäre Ammoniumsalze derselben.

3. Eine chemoetherapeutische Zusammensetzung enthaltend eine Verbindung der Formel I wie im Anspruch 1 oder 2 beansprucht, in Form der freien Base oder in Form eines chemotherapeutisch annehmbaren Säureadditions- oder quaternären Ammoniumsalzes, zusammen mit einen chemotherapeutisch annehmbaren Verdünnungsmittel oder Träger.

4. Eine Verbindung der Formel I wie im Anspruch 1 oder 2 beansprucht, in Form der freien Base oder in Form eines chemotherapeutisch annehmbaren Säureadditions- oder quaternären Ammoniumsalzes, zur Verwendung als chemotherapeutisches Mittel.

5. Eine Verbindung der Formel I wie im Anspruch 1 oder 2 beansprucht, in Form der freien Base oder in Form eines chemotherapeutisch annehmbaren Säureadditions- oder quaternären Ammoniumsalzes, zur Verwendung als anti-bakteriell wirksames Antibiotikum oder zur Behandlung von durch obligate Anaerobier verursachten Infectionen.

6. Verfahren zur Herstellung einer Verbindung der Formel I wie im Anspruch 1 dargelegt oder eines Säureadditions- oder quaternären Ammoniumsalzes derselben, bestehend aus Umsetzung einer Verbindung der Formel II

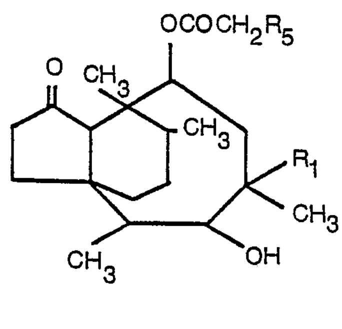

II

worin

$R_1$ wie oben definiert ist und

$R_5$ Chlor, Brom oder $OSO_2R_7$ bedeutet wobei $R_7$ für Alkyl oder Aryl steht mit einer Verbindung der Formel III

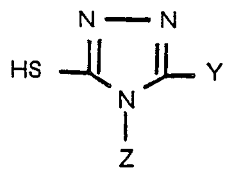

III

worin

Y und Z wie oben definiert sind,

und, gewünschtenfalls Umwandlung einer so erhaltenen Verbindung der Formel I in ein Säureadditions- oder quaternäres Ammoniumsalz derselben oder umgekehrt.

**Patentansprüche für den Vertragsstaat: AT**

1. Ein Verfahren zur Herstellung einer Verbindung der Formel I

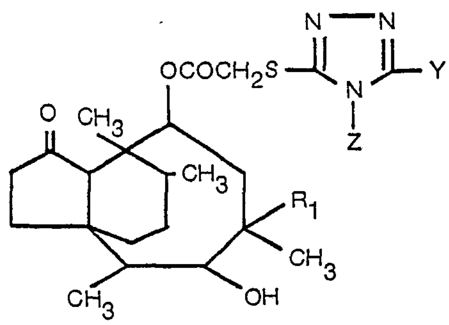

I

worin

$R_1$ Ethyl oder Vinyl;

Y Wasserstoff, Amino, Trifluormethyl, $C_{1-4}$-Alkyl, Pyridyl oder $—N(R_3)(R_4)$

Z Wasserstoff, $C_{1-4}$-Alkylsulfonyl, Amino, Formyl, $—N(R_3)(R_4)$, $—S(CH_2)_n—$, $—N(R_3)(R_4)$ oder $CX.NHR_6$; bedeuten,

$R_3$ und $R_4$ gleich oder verschieden sind und jeweils für Wasserstoff, $C_{1-4}$-Hydroxyalkyl, $C_{1-4}$-Dihydroxyalkyl, unsubstituiertes oder substituiertes $C_{1-4}$-Alkanoyl, $C_{1-4}$-Alkylsulfonyl oder $C_{1-4}$-Alkyl stehen, oder zusammen mit dem Stickstoffatom für einen Piperazinrest stehen der am zweiten Stickstoffatom durch $C_{1-4}$-Alkyl, $C_{1-4}$-Hydroxyalkyl oder $C_{1-4}$-Dihydroxyalkyl substituiert sein kann;

$R_6$ $C_{1-4}$-Alkyl oder $C_{1-4}$-Alkoxy-Carbonyl;

n 2 bis 5; und

X Sauerstoff oder Schwefel sind,

oder eines Säureadditions- oder quaternäre Ammoniumsalzes derselben bestehen aus Umsetzung einer Verbindung der Formel II

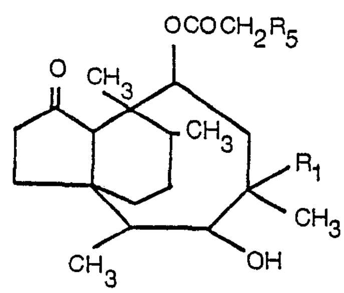

II

worin

$R_1$ wie oben definiert ist und

$R_5$ Chlor, Brom oder $OSO_2R_7$ bedeutet wobei $R_7$ für Alkyl oder Aryl steht mit einer Verbindung der Formel III

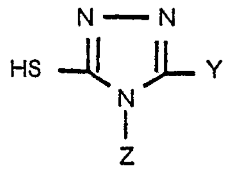

III

worin

Y und Z wie oben definiert sind,

und, gewünschtenfalls Umwandlung einer so erhaltenen Verbindung der Formel I in ein Säureadditions- oder quaternäres Ammoniumsalz derselben oder umgekehrt.

2. Verfahren wie im Anspruch 1 beansprucht worin die so erhaltene Verbindung 19,20-Dihydro-14-*O*-[3-amino-1,2,4-triazol-5-yl)-thioacetyl]mutilin oder ein Säureadditions- oder quaternäres Ammoniumsalz derselben ist.

3. Eine Verbindung der Formel I gemäss Anspruch 1 oder ein Säureadditions- oder ein quaternäres Ammoniumsalz derselben falls nach einem Verfahren wie im Anspruch 1 beansprucht hergestellt.

4. Chemotherapeutische Zusammensetzung enthaltend eine Verbindung der Formel I gemäss Anspruch 3, in Form des freien Base oder in Form eines chemotherapeutisch annehmbaren Säureadditions- oder quaternären Ammoniumsalzen zusammen mit einem chemotherapeutisch annehmbaren Verdünnungsmittel oder Träger.

5. Ein Verfahren zur Herstellung einer chemotherapeutischen Zusammensetzung welches darin besteht, dass man eine Verbindung der Formel I

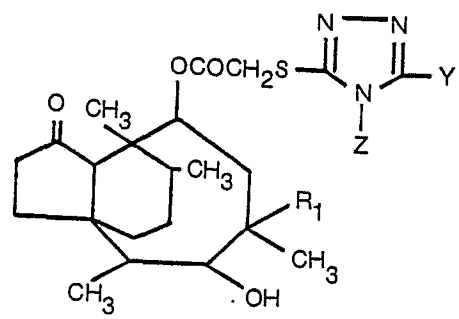

I

worin

$R_1$ Ethyl oder Vinyl;

Y Wasserstoff, Amino, Trifluormethyl, $C_{1-4}$-Alkyl, Pyridyl oder $—N(R_3)(R_4)$

Z Wasserstoff, $C_{1-4}$-Alkylsulfonyl, Amino, Formyl, $—N(R_3)(R_4)$, $—S(CH_2)_n—$, $—N(R_3)(R_4)$ oder $CX.NHR_6$; bedeuten,

$R_3$ und $R_4$ gleich oder verschieden sind und jeweils für Wasserstoff, $C_{1-4}$-Hydroxyalkyl, $C_{1-4}$-Dihydroxyalkyl, unsubstituiertes oder substituiertes $C_{1-4}$-Alkanoyl, $C_{1-4}$-Alkylsulfonyl oder $C_{1-4}$-Alkyl stehen, oder zusammen mit dem Stickstoffatom für einen Piperazinrest stehen der am zweiten Stickstoffatom durch $C_{1-4}$-Alkyl, $C_{1-4}$-Hydroxyalkyl oder $C_{1-4}$-Dihydroxyalkyl substituiert sein kann;

$R_6$ $C_{1-4}$-Alkyl oder $C_{1-4}$-Alkoxy-Carbonyl;

n 2 bis 5; und

X Sauerstoff oder Schwefel sind,

in Form der freien Base oder in Form eines chemotherapeutisch annehmbaren Säureadditions- oder quaternären Ammoniumsalzes zusammen mit einem chemotherapeutisch annehmbaren Verdünnungsmittel oder Träger vermischt.

**Revendications pour les Etats contractants: BE CH DE FR GB IT LU NL SE**

1. Un composé de formule I

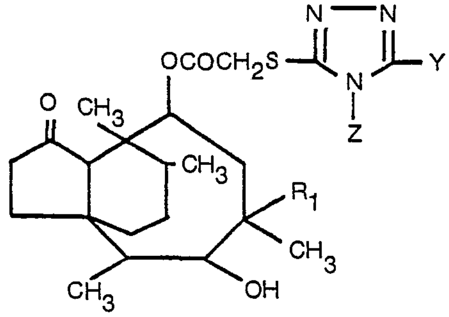

I

dans laquelle

$R_1$ est éthyle ou vinyle;

Y est hydrogène, amino, trifluorométhyle, $C_1$—$C_4$-alkyle, pyridyle ou —$N(R_3)(R_4)$;

Z est hydrogène, $C_1$—$C_4$-alkylsulfonyle, amino, formyle, —$N(R_3)(R_4)$, —$S(CH_2)_nN(R_3)(R_4)$ ou $CX.NHR_6$;

$R_3$ et $R_4$ sont identiques ou différents et chacun est hydrogène, $C_1$—$C_4$-hydroxyalkyle, $C_1$—$C_4$-dihydroxyalkyle, $C_1$—$C_4$-alcanoyle non substitué ou substitué, $C_1$—$C_4$-alkylsulfonyle ou $C_1$—$C_4$-alkyle ou ensemble avec l'atome d'azote représentent un radical pipérazinyle qui peut être substitué sur le second atome d'azote par $C_1$—$C_4$-alkyle, $C_1$—$C_4$-hydroxyalkyle ou $C_1$—$C_4$-dihydroxyalkyle;

$R_6$ est $C_1$—$C_4$-alkyle ou $C_1$—$C_4$-alcoxy-carbonyle,

n est 2 à 5; et

x est l'oxygène ou le soufre;

et ses sels d'addition d'acide et d'ammonium quaternaire.

2. 19,20-dihydro-14-O-[3-amino-1,2,4-triazole-5-yl)-thioacétyl)-mutiline et ses sels d'addition d'acide et d'ammonium quaternaire.

3. Une composition chimiothérapeutique comprenant un composé de formule I tel que revendiqué à la revendication 1 ou 2, sous forme de base libre ou sous la forme d'un sel d'addition d'acide ou d'ammonium quaternaire chimiothérapeutiquement acceptable, en association avec un diluant ou véhicule chimiothérapeutiquement acceptable.

4. Un composé de formule I tel que revendiqué à la revendication 1 ou 2, sous forme de base libre ou sous la forme d'un sel d'addition d'acide ou d'ammonium quaternaire chimiothérapeutiquement acceptable, pour l'utilisation comme agent chimiothérapeutique.

5. Un composé de formule I tel que revendiqué à la revendication 1 ou 2, sous forme de base libre ou sous la forme d'un sel d'addition d'acide ou d'ammonium quaternaire chimiothérapeutiquement acceptable, pour l'utilisation comme antibiotique à activité antibactérienne et pour le traitement des infections provoquées par des anaérobes obligatoires.

6. Un procédé de préparation d'un composé de formule I, indiqué à la revendication 1, ou d'un sel d'addition d'acide ou d'ammonium quaternaire de ce composé, qui comprend la réaction d'un composé de formule II

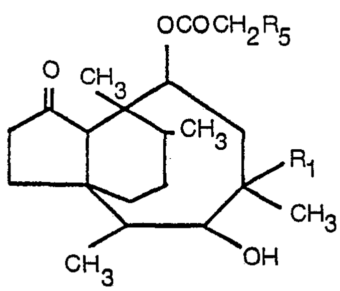

II

dans laquelle

$R_1$ est tel que défini à la revendication 1 et

$R_5$ est le chlore, le brome ou —$OSO_2R_7$, dans lequel $R_7$ est alkyle ou aryle,

avec un composé de formule III

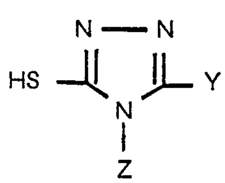

III

dans laquelle

Y et Z sont tels que définis à la revendication 1,

et, si désiré, la transformation d'un composé de formule I ainsi obtenu en un sel d'addition d'acide ou d'ammonium quaternaire de ce composé, ou vice versa.

**Revendications pour l'Etat contractant: AT**

1. Un procédé de préparation d'un composé de formule I

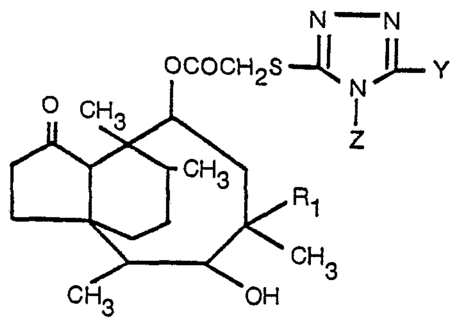

I

dans laquelle
$R_1$ est éthyle ou vinyle;
Y est hydrogène, amino, trifluorométhyle, $C_1$—$C_4$-alkyle, pyridyle ou —$N(R_3)(R_4)$;
Z est hydrogène, $C_1$—$C_4$-alkylsulfonyle, amino, formyle, —$N(R_3)(R_4)$, —$S(CH_2)_nN(R_3)(R_4)$ ou $CX.NHR_6$;
$R_3$ et $R_4$ sont identiques et différents et chacun est hydrogène, $C_1$—$C_4$-hydroxyalkyle, $C_1$—$C_4$-dihydroxyalkyle, $C_1$—$C_4$-alcanoyle non substitué ou substitué, $C_1$—$C_4$-alkylsulfonyle ou $C_1$—$C_4$-alkyle ou ensemble avec l'atome d'azote représentent un radical pipérazinyle qui peut être substitué sur le second atome d'azote par $C_1$—$C_4$-alkyle, $C_1$—$C_4$-hydroxyalkyle ou $C_1$—$C_4$-dihydroxyalkyle;
$R_6$ est $C_1$—$C_4$-alkyle ou $C_1$—$C_4$-alcoxycarbonyle;
n est 2 à 5; et
x est l'oxygène ou le soufre;
ou d'un sel d'addition d'acide ou d'ammonium quaternaire de ce composé, qui comprend la réaction d'un composé de formule II,

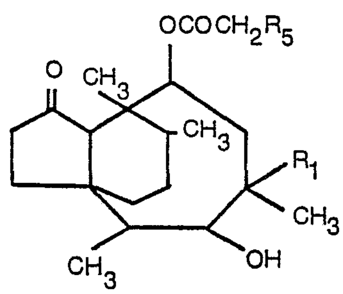

II

dans laquelle
$R_1$ est tel que défini ci-dessus, et
$R_5$ est le chlore, le brome ou —$OSO_2R_7$, dans lequel $R_7$ est alkyle ou aryle,
avec un composé de formule III,

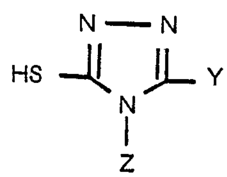

III

dans laquelle
Y et Z sont tels que définis ci-dessus, et, si désire, la transformation d'un composé de formule I ainsi obtenu en un sel d'addition d'acide ou d'ammonium quaternaire de ce composé, ou vice versa.

2. Un procédé tel que revendiqué à la revendication 1, dans lequel le composé ainsi obtenu est la 19,20-dihydro-14-O-[3-amino-1,2,4-triazole-5-yl)-thioacétyl]mutiline ou un sel d'addition d'acide ou d'ammonium quaternaire de ce composé.

3. Un composé de formule I selon la revendication 1 ou un sel d'addition d'acide ou d'ammonium quaternaire de ce composé, préparé selon un procédé tel que revendiqué à la revendication 1.

4. Une composition chimiothérapeutique comprenant un composé de formule I selon la revendication 3, sous forme de base libre ou sous la forme d'un sel d'addition d'acide ou d'ammonium quaternaire chimiothérapeutiquement acceptable, en association avec un diluant ou véhicule chimiothérapeutiquement acceptable.

5. Un procédé de préparation d'une composition chimiothérapeutique, qui comprend le mélange d'un composé de formule I

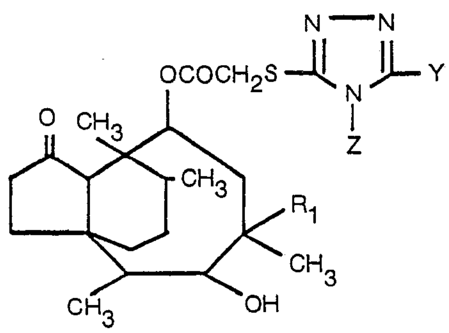

I

dans laquelle

$R_1$ est éthyle ou vinyle;

Y est hydrogène, amino, trifluorométhyle, $C_1$—$C_4$-alkyle, pyridyle ou —$N(R_3)(R_4)$;

Z est hydrogène, $C_1$—$C_4$-alkylsulfonyle, amino, formyle, —$N(R_3)(R_4)$, —$S(CH_2)_nN(R_3)(R_4)$ ou $CX.NHR_6$;

$R_3$ et $R_4$ sont identiques ou différents et chacun est hydrogène, $C_1$—$C_4$-hydroxyalkyle, $C_1$—$C_4$-dihydroxyalkyle, $C_1$—$C_4$-alcanoyle non substitué ou substitué, $C_1$—$C_4$-alkylsulfonyle ou $C_1$—$C_4$-alkyle ou ensemble avec l'atome d'azote représentent un radical pipérazinyle qui peut être substitué sur le second atome d'azote par $C_1$—$C_4$-alkyle, $C_1$—$C_4$-hydroxyalkyle ou $C_1$—$C_4$-dihydroxyalkyle;

$R_6$ est $C_1$—$C_4$-alkyle ou $C_1$—$C_4$-alcoxy-carbonyle;

n est 2 à 5; et

x est l'oxygène ou le soufre;

sous forme de base libre ou sous la forme d'un sel d'addition d'acide ou d'ammonium quaternaire chimiothérapeutiquement acceptable, ensemble avec un diluant ou véhicule chimiothérapeutiquement acceptable.